(19) Europäisches Patentamt
European Patent Office
Office européen des brevets

(11) Veröffentlichungsnummer: **0 561 237 A2**

# (12) EUROPÄISCHE PATENTANMELDUNG

(21) Anmeldenummer: **93103583.6**

(51) Int. Cl.[5]: **C07C 217/84**, A61K 31/135

(22) Anmeldetag: **05.03.93**

(30) Priorität: **18.03.92 US 853196**

(43) Veröffentlichungstag der Anmeldung:
**22.09.93 Patentblatt 93/38**

(84) Benannte Vertragsstaaten:
**AT BE CH DE DK ES FR GB GR IE IT LI LU MC NL PT SE**

(71) Anmelder: **BAYER AG**

**D-51368 Leverkusen(DE)**

(72) Erfinder: **Lenfers, Jan-Bernd, Dr.**
**Hubertusallee 9**
**W-5600 Wuppertal 1(DE)**
Erfinder: **Muschalek-Letina, Volker, Dr.**
**Claudiusweg 3**
**W-5600 Wuppertal 1(DE)**
Erfinder: **Niermers, Ekkehard, Dr.**
**In den Birken 51a**
**W-5600 Wuppertal 1(DE)**
Erfinder: **Scriabine, Alexander, Prof. Dr.**
**435 Colonial Road**
**Guilford, CT 06437(US)**
Erfinder: **Chisholm, Jane, Dr.**
**66 Killingworth**
**Turnpike, Clinton, CT 06413(US)**
Erfinder: **Hunnicutt, Edward, Dr.**
**42 Jerome Lane**
**Milford, CT 06460(US)**

(54) **Enantiomer, (-)-N-(2-Ethoxyphenyl)-N'-(1,2,2-trimethylpropyl)-2-nitromethen-1,1-diamin, Verfahren zu dessen Herstellung und Verwendung desselben.**

(57) Die Erfindung betrifft das Enantiomer (-)-N-(2-Ethoxyphenyl)-N'-(1,2,2-trimethylpropyl)-2-nitroethen-1,1-diamin, ein Verfahren zu dessen Herstellung und die Verwendung desselben als Medikament, das nervöse und neuro-endokrine Erkrankungen, darunter zerebrale Ischämie, Reizschädigungen, Hirnschlag, Demenzen und Epilepsie beeinflußt, ohne jedoch auf diese beschränkt zu sein. Potentielle Verwendungen bei höheren Konzentrationen schließen Angina, Hypertonie, Harninkontinenz, Haarausfall, Asthma, periphere Gefäßerkrankungen, Herzrhythmusstörungen, Myokardischämie, Sexualdysfunktion und Entzündungen ein.

EP 0 561 237 A2

Die vorliegende Erfindung betrifft das Enantiomer (-)-N-(2-Ethoxyphenyl)-N'-(1,2,2-trimethylpropyl)-2-nitroethen-1,1-diamin, ein Verfahren zu dessen Herstellung und die Verwendung desselben als Medikament, das das Nerven- und Nerven-Hormon-Gewebe beeinflußt, ohne sich jedoch auf diese zu beschränken, insbesondere als Mittel zur Verwendung bei pathologischen Zuständen des ZNS, darunter Hirnschlag, Ischämie, Epilepsie und Demenzen. Potentielle Verwendungen bei höheren Konzentrationen schließen Angina, Hypertonie, Harninkontinenz, Haarausfall, Asthma, peripheren Gefäßerkrankungen, Herzrhythmusstörungen, Myokardischämie, Sexualdysfunktion und Entzündungen ein.

Die pharmakologischen Wirkungen eines racemischen Gemischs, das sowohl das (+)- als auch das (-)-Enantiomer enthält, werden manchmal nur durch das eine der zwei Enantiomeren hervorgerufen, während das andere Enantiomer wenig oder gar keine Aktivität besitzt. Im vorliegenden Fall zeigt das (-)-Enantiomer potente Effekte, nicht aber das (+)-Enantiomer.
Tatsächlich kann das (+)-Enantiomer die potente und nutzbringende Aktivität des (-)-Enantiomers blockieren.

Zusätzlich zeigt das (+)-Enantiomer, jedoch nicht das (-)-Enantiomer, eine bevorzugte Aktivität an der glatten Muskulatur {z.B. beschreibt die DE 32 32 462 das racemische Gemisch N-(2-Ethoxyphenyl)-N'-(1,2,2-trimethylpropyl)-2-nitroethen-1,1-diamin, das eine Wirkung auf die Gefäßmuskulatur ausübt}. Das (-)-Enantiomer ist auch neuroprotektiv wirksam mit einer Konzentration von 1/10 000 derjenigen, die für die Aktivität des (+)-Enantiomers erforderlich ist. Somit ist das therapeutische Potential des (-)-Enantiomers "einzigartig" im Vergleich zu dem des racemischen Gemischs.

Beim Vergleich des erfindungsgemäßen (-)-Enantiomers mit dem (+)-Enantiomer ist ein pharmakologisch unterschiedlicher Mechanismus zu erkennen. Das (+)-Enantiomer kehrt die Depolarisierung unter den Bedingungen der Absenkung des intrazellulären ATP-Spiegels um, d. h., es ist ein durch Glyburide inhibierbares Hyperpolarisierungsmittel, das in Zellen und Geweben aktiv ist, die ATP-abhängige K-Kanäle besitzen, und wird dementsprechend als ein $K_{ATP}$-Kanal-Öffner angesehen. Im Gegensatz dazu zeigt das (-)-Enantiomer eine hyperpolarisierende Wirkung, die durch Glyburide nicht blockiert wird. Es ist in einem Zell-Typ (der MIPP-N1E-115-Neuroblastomlinie) wirksam, der nicht auf das (+)-Enantiomer anspricht, und ist unwirksam in einer Zellinie des glatten Muskels. Ebenfalls bemerkenswert ist die hohe Potenz der erfindungsgemäßen Verbindung (mit einem $EC_{50}$ <10 picomolar bei dieser neu aufgefundenen Aktivität).

Die Potenz der erfindungsgemäßen Verbindung ist etwa 1 000 bis 10 000mal höher als die Potenz des (+)-Enantiomers, gemessen in den für die hyperpolarisierende Aktivität erforderlichen Konzentrationen. Dies zeigt eine hohe Spezifität und einen hohen therapeutischen Index an.

Die erfindungsgemäße Verbindung zeigt eine bemerkenswerte Neuroprotektion, die an einzelnen Neuronen aus dem Zentralnervensystem von Ratten demonstriert werden kann. Die hyperpolarisierende Aktivität in Neuronen vom Typ der Pyramidenzellen aus dem Hippocampus mit der Hemmung erhöhter Calcium-Konzentrationen während der Langzeit-Depolarisierung zeigt diese unerwartete Eigenschaft, weil diese Neuronen dafür bekannt sind, daß sie bei verschiedenen pathologischen Zuständen in Verbindung mit einer Calcium-Überlastung selektiv geschädigt werden. Die neuroprotektive Wirkung is hilfreich gegen die Degeneration während Perioden der Reiztoxizität und der pathologischen Depolarisierung.

Die Erfindung betrifft das Enantiomer (-)-N-(2-Ethoxyphenyl)-N'-(1,2,2-trimethylpropyl)-2-nitroethen-1,1-diamin der Formel (I)

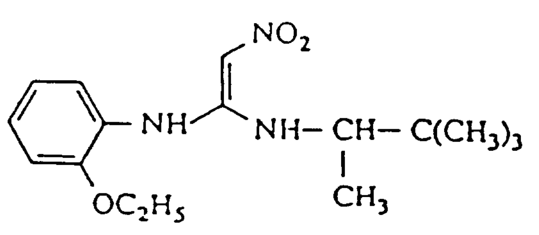

(I)

(-)-Enantiomer

Die erfindungsgemäße Verbindung ist neu und besitzt wertvolle pharmakologische Eigenschaften. Überraschenderweise zeichnet sie sich durch eine außerordentliche starke, das Neuronen-Gewebe beeinflussende Wirkung aus.

Die erfindungsgemäße Verbindung der Formel (I) kann dadurch hergestellt werden, daß
[A] 1-[(1-Ethoxy)phenylamino]-1-methylthio-2-nitroethen der Formel (II)

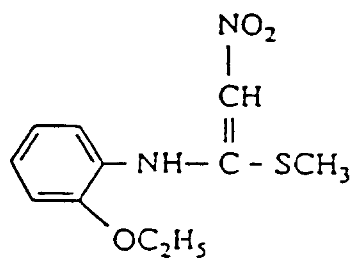

(II)

mit 1,2,2-Trimethylpropylamin der Formel (III)

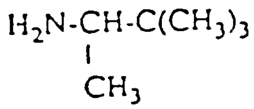

(III)

umsetzt, gegebenenfalls in Gegenwart eines organischen Lösungsmittels, um das entsprechende racemische Gemisch zu bilden, und anschließend das (-)-Enantiomer direkt an einer chiralen stationären Phase mittels Hochleistungs-Flüssigkeits-Chromatographie (HPLC) und organischer Lösungsmittel aus dem racemischen Gemisch abtrennt,
oder
[B] das enantiomer reine 1,2,2-Trimethylpropylamin der Formel (IIIa)

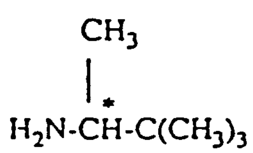

(IIIa)

mit 1-[(1-Ethoxy)phenylamino]-1-methylthio-2-nitroethen der Formel (II) nach der unter [A] angebenen Verfahrensweise umsetzt.

Das Verfahren kann beispielhaft anhand des folgenden Formel-Diagramms veranschaulicht werden:

[A]

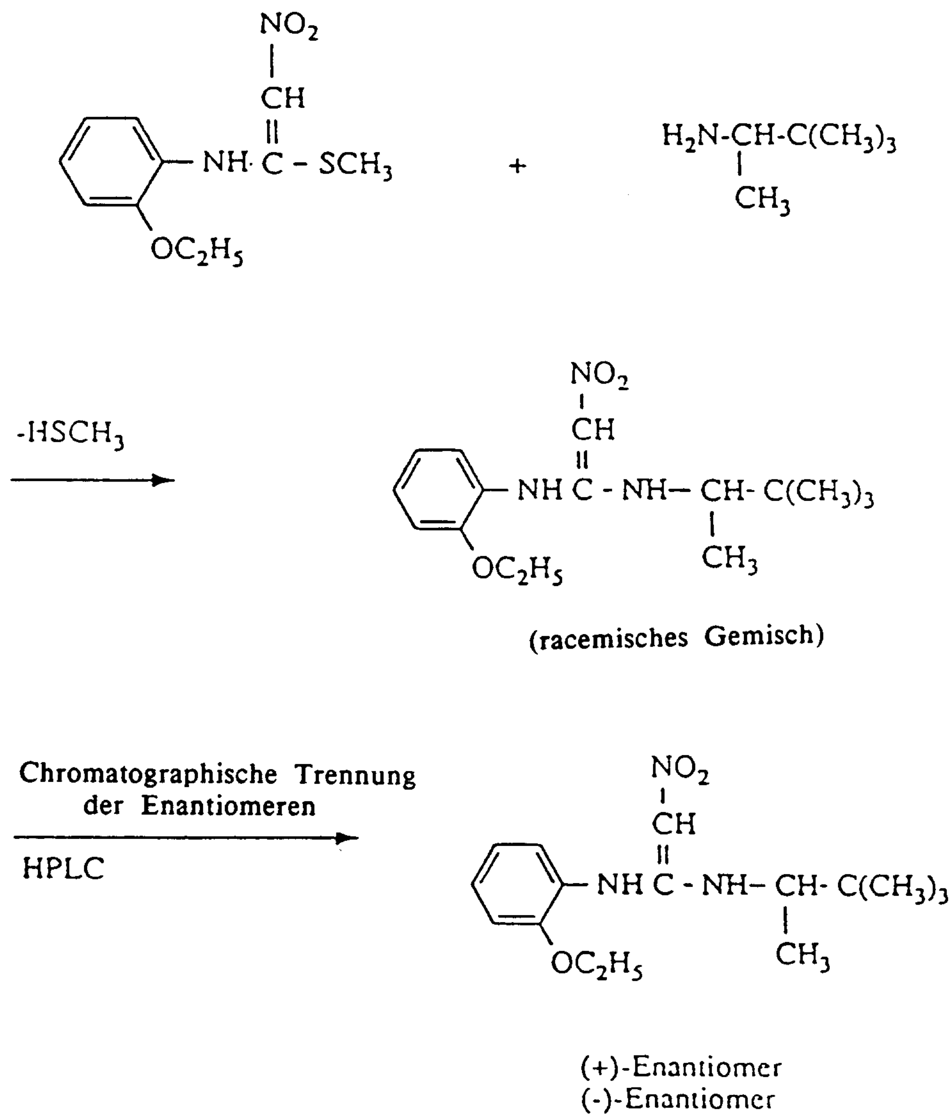
-HSCH3
(racemisches Gemisch)
Chromatographische Trennung der Enantiomeren
HPLC
(+)-Enantiomer
(-)-Enantiomer

[B]

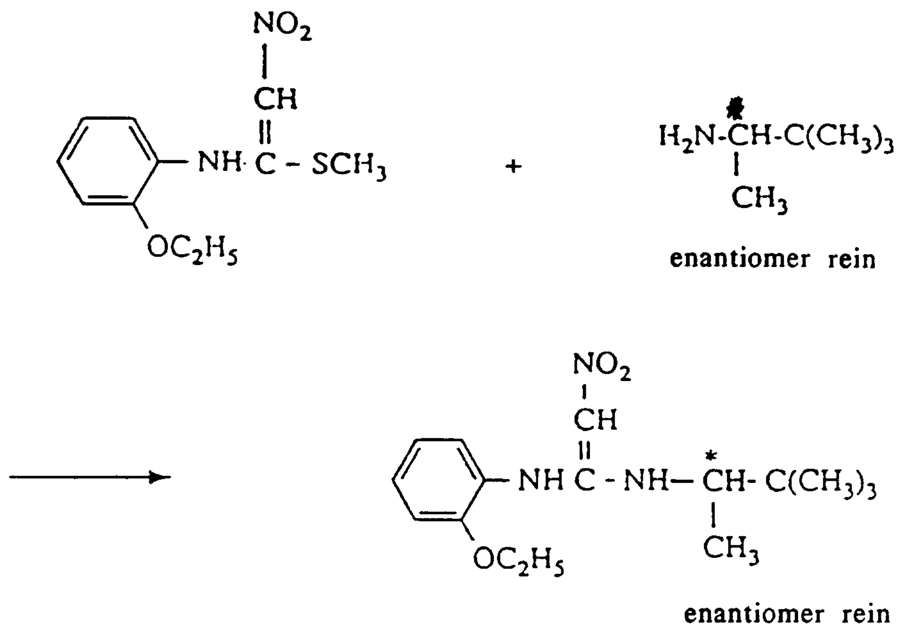


Die Herstellung des racemischen Gemischs ist in DE 32 32 462 beschrieben.

In dem Fall, in dem die Reaktion in Anwesenheit eines organischen Lösungsmittels durchgeführt wird, sind geeignete organische Lösungsmittel Kohlenwasserstoffe wie Hexan, Toluol und Xylol, oder chlorierte Kohlenwasserstoffe wie Methylenchlorid und Chloroform, oder Ether wie Diethylether, Dioxan und Tetrahydrofuran, oder Alkohole wie Ethanol, Ethylenglycol oder Methanol, und Eisessig, Pyridin, Dimethylsulfoxid oder Dimethylformamid. Es ist auch möglich, die Reaktion ohne ein Lösungsmittel durchzuführen, jedoch mit einem Überschuß des Amins, 1,2,2-Trimethylpropylamin. Die Verwendung von Eisessig, Ethanol und des Amin-Überschusses wird bevorzugt.

Die Reaktion wird in einem Temperaturbereich von 50 °C bis 160 °C, vorzugsweise von 50 °C bis 120 °C, durchgeführt.

Die chemische Reaktion wird im allgemeinen unter Atmosphärendruck durchgeführt. Die HPLC-Trennung wird unter einem Überdruck von 10 bis 25 bar durchgeführt.

Die Verbindung der Formel (III) wird in einer Menge von 1 bis 5 mol, vorzugsweise von 1 bis 3 mol, relativ zu 1 mol der Verbindung (II), eingesetzt.

Bei der HPLC-Trennung werden sowohl der präparative als auch der analytische Arbeitsgang mit einer Phase durchgeführt, die aus Cellulose-tris-3,5-dimethylphenylcarbamat auf Silicagel besteht, Markenbezeichnung Chiracel OD.

Geeignete Lösungsmittel sind in diesem Zusammenhang Kohlenwasserstoffe wie n-Heptan und n-Hexan oder Alkohole wie 2-Propanol. Es ist auch möglich, Gemische dieser Lösungemittel zu verwenden. Bevorzugte Gemische sind 78 : 22 n-Hexan/2-Propanol für den präparativen und 75 : 25 n-Hexan/2-Propanol für den analytischen Teil der Chromatographie.

Die HPLC wird bei 20 °C bis 60 °C, vorzugsweise bei 40 °C, durchgeführt.

Ausgehend von dem racemischen Gemisch ist die Fraktionierung in die stereochemisch einheitlichen Bestandteile, insbesondere des erfindungsgemäßen (-)-Enantiomers, im Prinzip auch mit Hilfe anderer Verfahren möglich, die aus der Literatur bekannt sind (vgl. E.L. Eliel, Stereochemistry of Carbon Compounds, McGraw Hill, 1962).

Die Verbindungen der Formeln (II), (III) und (IIIa) sind bekannt und können mit Hilfe von Verfahren hergestellt werden, die aus der Literatur bekannt sind (Chem. Ber. 100, 591-604 (1967); Houben-Weyl XI/1 (1957); H.E. Smith, E.E. Ensley, Can. J. of Chem. 49, 1971, 2902).

Die erfindungsgemäße Verbindung ist potentiell bei der Behandlung von Patienten nützlich, die an Hirnschlag, Ischämie oder Demenzen leiden. Sie kann auch bei der Laboratoriumsdiagnose solcher

Zustände von Nutzen sein, d.h. bei Tests zur Bestimmung des Zustandes des Patienten.

Die neue aktive Verbindung kann in bekannter Weise in übliche Formulierungen überführt werden, etwa in Tabletten, beschichtete Tabletten, Pillen, Granulat, Aerosole, Sirupe, Emulsionen, Suspensionen und Lösungen, wobei inerte, nicht-toxische, pharmazeutisch geeignete Streckmittel oder Lösungsmittel zum Einsatz kommen. In diesem Zusammenhang sollte die therapeutisch aktive Verbindung in jedem Falle in einer Konzentration von etwa 0,5 bis 90 Gew.-% der gesamten Mischung vorliegen, d.h. in solchen Mengen, die zur Erzielung des angegebenen Dosis-Bereichs ausreichend sind.

Die Formulierungen werden beispielsweise dadurch hergestellt, daß die aktiven Verbindungen mit Lösungsmitteln und/oder Streckmitteln verschnitten werden, gegebenenfalls unter Verwendung von Emulgatoren und Dispergiermitteln, wobei es beispielsweise im Fall der Verwendung von Wasser als Verdünnungsmittel möglich ist, gegebenenfalls organische Lösungsmittel als Hilfslösungsmittel zu verwenden.

Die Verabreichung sollte in üblicher Weise erfolgen, vorzugsweise oral oder parenteral, insbesondere perlingual oder intravenös.

Im Fall der parenteralen Verabreichung sollten Lösungen der aktiven Verbindung unter Einsatz geeigneten flüssiger Streckmittel zur Anwendung kommen.

Im allgemeinen erweist es sich als vorteilhaft, intravenöse Mengen, bezogen auf das Körpergewicht, von 0,001 bis 1 mg/kg, vorzugsweise von 0,01 bis 0,5 mg/kg, zu verabreichen, um wirksame Ergebnisse zu erzielen, und bei der oralen Verabreichung beträgt die Dosis etwa 0,01 bis 20 mg/kg, vorzugsweise 0,1 bis 10 mg/kg, bezogen auf das Körpergewicht.

Trotzdem kann es notwendig sein, von den genannten Mengen abzuweichen, was speziell vom Körpergewicht oder der Art und Weise der Verabreichung, dem individuellen Verhalten gegenüber dem Medikament, der Art und Weise seiner Formulierung und dem Zeitpunkt oder der Zeitspanne, zu dem bzw. während der die Verabreichung stattfindet, abhängt. So kann es in manchen Fällen ausreichend sein, mit weniger als der zuvor erwähnten minimalen Menge zu arbeiten, während in anderen Fällen der genannte obere Grenzwert überschritten werden muß. Im Fall der Verabreichung relativ großer Mengen kann es ratsam sein, diese in eine Anzahl von Einzel-Dosierungen über den Verlauf des Tages hinweg aufzuteilen.

Das racemische Gemisch (-/+)-N-(2-Ethoxyphenyl)-N'-(1,2,2-trimethylpropyl)-2-nitroethen-1,1-diamin, dessen Herstellung in DE 32 32 462 beschrieben ist, wird an einer chiralen stationären Phase direkt mittels Hochleistungs-Flüssigkeits-Chromatographie (HPLC) getrennt.

Verwendete Materialien

1. Stationäre Phase:
Für präparative Arbeiten wird Cellulose-tris-3,5-dimethylphenylcarbamat an Silicagel, Markenbezeichnung Chiracel OD, erhältlich von J.T. Baker {Teilchengröße: 20 µm, Porenweite 100 nm (1 000 Å)}, adsorbiert. Das Material wird mit Hilfe der Aufschlämmungstechnik in die Säule gepackt. Die analytischen Arbeiten wurden an der oben angegebenen Phase durchgeführt. Die vorher gepackte Säule (Teilchengröße 10 µm) wurde von J.T. Baker bezogen.

2. Lösungsmittel:
n-Heptan, analysenrein (Merck)
n-Hexan, analysenrein (Merck)
2-Propanol, analysenrein (Merck)

Proben-Vorbereitung

300 mg des racemischen Gemischs (-/+)-N-(2-Ethoxyphenyl)-N'-(1,2,2-trimethylpropyl)-2-nitroethen-1,1-diamin werden in 40 ml 2-Propanol gelöst.

Chromatographie-Bedingungen

| präparativ | | |
|---|---|---|
| Säule: | Länge:<br>Innendurchmesser: | 550 mm<br>20 mm |
| Fließgeschwindigkeit: | | 10 ml/min |
| Mobile | Phase: | 78/22 n-Hexan/2-Propanol |
| Wanderungszeit einer Injektion: | | 45 min |
| Nachweis: | | UV-VIS-Detektor<br>Wellenlänge: 330 nm |
| Temperatur: | | 40 °C |
| Aufgetragenes Volumen: | | 1 ml = 7,5 mg |

| analytisch | | |
|---|---|---|
| Säule: | Länge:<br>Innendurchmesser: | 250 mm<br>4,6 mm |
| Mobile Phase: | | 75/25 n-Heptan/2-Propanol |
| Fließgeschwindigkeit: | | 1 ml/min |
| Nachweis: | | UV-VIS-Detektor<br>Wellenlänge: 230 nm |
| Temperatur: | | 40 °C |
| Aufgetragenes Volumen: | | 20 µl |

Beispiel 1

(-)-N-(2-Ethoxyphenyl)-N'-(1,2,2-trimethylpropyl)-2-nitroethen-1,1-diamin

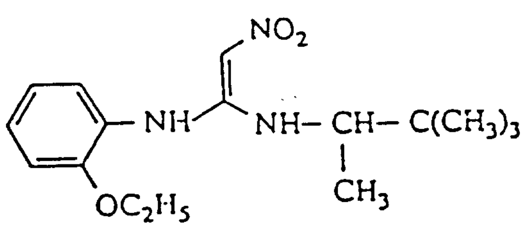

(-)-Enantiomer

Ausbeute: 100 mg
Enantiomeren-Überschuß > 99 %
$[\alpha]_D^{20}$ = -61,3° (c = 0,9; $CH_2Cl_2$).
Retentionszeit: 14,3 min.

Beispiel 2 (Vergleich)

(+)-N-(2-Ethoxyphenyl)-N'-(1,2,2-trimethylpropyl)-2-nitroethen-1,1-diamin

Ausbeute: 100 mg
Enantiomeren-Überschuß > 99 %
$[\alpha]_D^{20} = +63{,}1°$ (c = 0,94; $CH_2Cl_2$).
Retentionszeit: 13,3 min.

Der $IC_{50}$-Wert des (-)-Enantiomers aus Beispiel 1 beträgt 5 pM in Neuronen.

Das entsprechende (+)-Enantiomer (Beispiel 2) ist nur bei mehr als dem 10 000-fachen dieser Konzentration, mit einem $EC_{50}$-Wert von 500 nM, wirksam. Außerdem ist das (+)-Enantiomer bei Vaskulargewebe ($IC_{50}$ = 15 nM, A10-Zellen) wirksamer als das (-)-Enantiomer ($IC_{50}$ = 250 nM jeweils). Schließlich wird die potente Wirkung von 5 pM des (-)-Enantiomers auf Neuronen durch das (+)-Enantiomer (1 μM) blockiert. Wegen der hohen Spezifität der Wirkung des (-)-Enantiomers und der Fähigkeit des (+)-Enantiomers, diese Wirkung zu blockieren, sollte Patienten mit nervösen Störungen nur das (-)-Enantiomer verabreicht werden.

**Patentansprüche**

1. (-)-N-(2-Ethoxyphenyl)-N'-(1,2,2-trimethylpropyl)-2-nitroethen-1,1-diamin der Formel

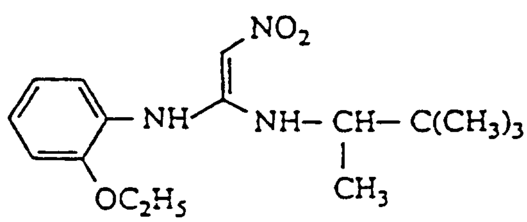

(I)

das im wesentlichen von dem (+)-Enantiomer frei ist.

2. Verwendung der Verbindung nach Anspruch 1 zur Herstellung einer Zusammensetzung zur Bekämpfung nervöser oder neuro-endokriner Erkrankungen.

3. Verwendung der Verbindung nach Anspruch 1 zur Herstellung von Zusammensetzungen zur Behandlung von Reiztoxizität, Hirnschlag, zerebraler Ischämie oder Demenz.

4. Pharmazeutisch wirksame Zusammensetzung, umfassend eine pharmazeutisch wirksame Menge der Verbindung nach Anspruch 1 und ein pharmakologisch verträgliches Verdünnungsmittel.

5. Verfahren zur Herstellung von (-)-N-(2-Ethoxyphenyl)-N'-(1,2,2-trimethylpropyl)-2-nitroethen-1,1-diamin der Formel

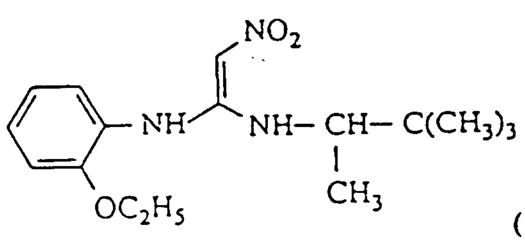

(I)

(-)-Enantiomer

umfassend
[a] die Reaktion von 1-[(1-Ethoxy)phenylamino]-1-methylthio-2-nitroethen der Formel

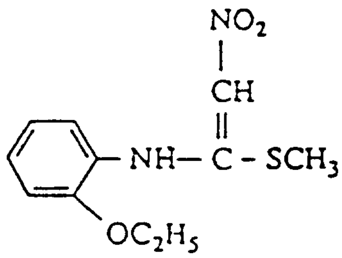

(II)

mit 1,2,2-Trimethylpropylamin der Formel

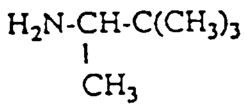

(III)

zu dem entsprechenden racemischen Gemisch und anschließend die direkte Abtrennung des (-)-Enantiomers an einer chiralen stationären Phase mittels Hochleistungs-Flüssigkeits-Chromatographie (HPLC) mit einem organischen Lösungsmittel,

oder

[b] die Reaktion von enantiomer reinem 1,2,2-Trimethylpropylamin der Formel

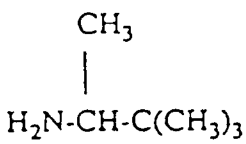

(IIIa)

enantiomerenrein

mit 1-[(1-Ethoxy)phenylamino]-1-methylthio-2-nitroethen.

**6.** Verfahren nach Anspruch 5, dadurch gekennzeichnet, daß die Reaktion in einem Temperatur-Bereich von 50 °C bis 160 °C durchgeführt wird.